# EUROPEAN PATENT APPLICATION

(11) **EP 4 483 786 A1**
(43) Date of publication of application: **01.01.2025**
(21) Application number: 23193668.3
(22) Date of filing: 28.08.2023
(51) Int. Cl.: A61B 5/00, A61B 5/117

(54) **FETAL MONITORING SYSTEM**

(30) Priority: 27.06.2023 US 202363523437 P
(71) Applicant: Koninklijke Philips N.V., 5656 AG Eindhoven (NL)
(72) Inventor: MEFTAH, Mohamed, Eindhoven (NL); HAMELMANN, Paul Christoph, Eindhoven (NL)
(74) Representative: Philips Intellectual Property & Standards

(57) **Abstract**

A fetal monitoring system including at least two electrodes, a user interface device with at least one input sensor, and a processor is provided. The electrode(s) are arranged on a user and capture two or more electrode signals. The input sensor(s) capture an input sensor signal. The processor (1) generates fetal movement detection data based on the input sensor signal; (2) generates eCTG data based on the electrode signals; and (3) generates a fetal monitoring dashboard displaying the fetal movement detection data and the eCTG data in a time-aligned manner. The eCTG data may include fetal heart rate data and/or uterine activity data. The fetal movement detection data may be generated further based on an input reference signal unique to the user. The fetal movement detection data may be further based on a differential signal generated corresponding to a maternal electrocardiogram signal.

## Description

### FIELD OF THE INVENTION

The present disclosure is generally directed to a fetal monitoring system, more particularly, to systems and methods for capturing and tracking fetal movement.

### BACKGROUND OF THE INVENTION

Cardiotocography (CTG) is the simultaneous monitoring of fetal heart rate and uterine contractions. CTG is a well-established method to evaluate fetal well-being in the antepartum and intrapartum pregnancy stages. A standard method for obtaining a cardiotocogram involves the use of (1) an ultrasound doppler transducer to detect fetal heart rate and (2) a tocodynamometer to measure uterine contractions. In addition, the doppler ultrasound transducer may also enable measuring fetal movements.

Providers of CTG fetal monitors have recently brought new CTG monitoring devices to the market. These new CTG monitoring devices are based on an alternative technique of electrophysiology based CTG (eCTG). eCTG solutions have the potential to provide more accurate fetal heart rate information, work better in high body-mass-index (BMI) patients, and provide a higher level of comfort for pregnant mothers. In addition, eCTG solutions do not require frequent repositioning, as is required for ultrasound Doppler based CTG fetal monitors. These properties make eCTG the technology of choice for remote fetal monitoring solutions.

A drawback of these eCTG solutions is that they are unable to monitor fetal movement. Presence of fetal movement during pregnancy is a key indicator for fetal well-being. Usually, mothers feel their babies for the first time around 16 to 20 weeks of gestation. After that, frequency, intensity, duration, and type of fetal movements changes throughout pregnancy. Unusual patterns of fetal movements in the antepartum period are often an early indicator of fetal compromise and, therefore, it is often advised to monitor fetal movements quantitatively.

Currently there is no integrated solution available that enables fetal movement tracking in electrophysiology based CTG monitoring systems. Existing electrophysiology-based fetal monitoring products can monitor fetal heart rate, maternal heart rate, and uterine activity, but they cannot track fetal movement. Fetal movements may be directly derived from electrophysiological signals. However, this only allows identification of gross-body movement, as the method relies on the relative location of the fetal heart with respect to the measurement electrodes. Hence, it is not possible to detect fetal kicks with this method. Accordingly, there is a need in the art for an eCTG solution capable of tracking fetal movement.

### SUMMARY OF THE INVENTION

The invention is defined by the independent claims. Advantageous embodiments are defined in the dependent claims.

The present disclosure is generally directed to a fetal monitoring system, and more particularly, to systems and methods for capturing and tracking fetal movement. The system is typically embodied as a wearable device with two or more electrodes, a user interface device with at least one input sensor used to track fetal movement, and a processor. The two or more electrodes are arranged on the user. The user interface device is configured to enable a user to interact with or activate the input sensor upon feeling or observing fetal movement. In some instances, the user may visually observe fetal movement (such as fetal kicks on the abdomen) without feeling the fetal movement. The input sensors could be electrodes, capacitive bioelectric sensors, microphones, fingerprint sensors, etc. The processor uses a detection algorithm to generate fetal movement detection data used to track fetal movement based on signals captured by the input sensor. The processor also uses additional detection algorithms to detect additional electrophysiology-based cardiotocography (eCTG) data, such as fetal heart rate and/or uterine activity. The processor then synchronizes and combines the fetal movement data and the eCTG data into a time-aligned dashboard for visualization on a display screen.

An advantage of synchronizing and combining the fetal movement data and the eCTG data is enabling easy eCTG data interpretation by a healthcare practitioner.

In some examples, the detection algorithm compares the signals captured by the input sensor(s) to an input reference signal to determine if the user is attempting to track fetal movement. In some cases, the processor may be trained to generate defined user-specific input reference signals according to training signals. In other instances, the input reference signal may be a stock, generic signal. For example, if the input sensor is a microphone, the input reference signal may correspond to a specific word or phrase spoken by the particular user.

In some examples, the processor generates a differential signal corresponding to a maternal electrocardiogram (ECG) signal of the user based on signals captured by one of the electrodes and the input sensor. The processor then uses a detection algorithm to generate fetal movement detection data used to track fetal movement based on the differential signal. In some cases, the detection algorithm compares the differential signal to a predefined ECG signal to generate fetal movement detection data. In other cases, the detection algorithm analyzes the differential signal for known patterns corresponding to fetal movement detection. In some cases, the processor may be trained to generate defined user-specific ECG reference signals according to training differential signals. In other instances, the predefined ECG signal may be a stock, generic ECG signal. In other cases, the detection algorithm may analyze the differential signal for characteristics representative of an ECG signal. For example, the detection algorithm may utilize a Pan-Tompkins method to detect QRS complexes. In other examples, the detection algorithm may rely on ECG wavelet transforms or other decomposition techniques.

In some examples, one or more additional detection algorithms may be used to generate maternal heart rate data based on one or more signals captured by the electrodes. The maternal heart rate data may be visualized on the fetal monitoring dashboard along with the fetal movement data and the eCTG data in a time-aligned manner.

In some examples, the input sensor is a dry electrode configured to be touched by the user when they feel fetal movement. In other examples, the input sensor is a capacitive bioelectric sensor which does not require direct skin contact to measure ECG signals. In addition to tracking instances of fetal movement, the fetal movement data may also track movement time, movement duration, and movement intensity based on the interaction of the user with the input sensor.

Generally, in one aspect, a fetal monitoring system is provided. The fetal monitoring system includes at least two electrodes. The at least two electrodes are configured to be arranged on a user. The at least two electrodes are further configured to capture two or more electrode signals.

The fetal monitoring system further includes a user interface device. The user interface device includes at least one input sensor. The at least one input sensor is configured to capture an input sensor signal. The at least one input sensor may be an additional electrode, a capacitive bioelectric sensor, a microphone, or a fingerprint sensor.

The fetal monitoring system further includes a processor. The processor is configured to generate fetal movement detection data based on the input sensor signal. The fetal movement detection data may be generated further based on a differential signal. The differential signal may be generated based on the input sensor signal and one of the two or more electrode signals. The differential signal may be a maternal electrocardiogram signal corresponding to the user. The fetal movement detection data may include movement time, movement duration, and movement intensity.

The processor is further configured to generate eCTG data based on the two or more electrode signals. The eCTG data may include fetal heart rate data and/or uterine activity data.

The processor is further configured to generate a fetal monitoring dashboard. The fetal monitoring dashboard is configured to display the fetal movement detection data and the eCTG data in a time-aligned manner.

According to an example, the fetal movement detection data may be generated further based on an input reference signal. The input reference signal may be a predefined electrocardiogram signal. According to a further example, the input reference signal is unique to the user. The processor is further configured to calibrate the input reference signal according to one or more training signals.

According to an example, the processor is further configured to generate maternal heart rate data. The maternal heart rate data is based on the two or more electrode signals. The fetal monitoring dashboard is further configured to display the maternal heart rate data.

According to an example, the fetal monitoring system further includes a display screen configured to display the fetal monitoring dashboard.

Generally, in another aspect, a method for fetal monitoring is provided. The method includes capturing, via two or more electrodes arranged on a user, two or more electrode signals.

The method further includes capturing, via at least one input sensor of a user interface device, an input sensor signal.

The method further includes generating, via a processor, fetal movement detection data based on the input sensor signal. The fetal movement detection data may be generated further based on an input reference signal.

The method further includes generating eCTG data based on the two or more electrode signals.

The method further includes generating a fetal monitoring dashboard configured to display the fetal movement detection data and the eCTG data in a time-aligned manner.

According to an example, the method further includes calibrating, via the processor, the input reference signal according to one or more training signals.

According to an example, the method further includes displaying, via a display screen, the fetal monitoring dashboard.

In various implementations, a processor or controller may be associated with one or more storage media (generically referred to herein as "memory," e.g., volatile and non-volatile computer memory such as RAM, PROM, EPROM, EEPROM, floppy disks, compact disks, optical disks, magnetic tape, SSD, etc.). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects as discussed herein. The terms "program" or "computer program" are used herein in a generic sense to refer to any type of computer code (e.g., software or microcode) that can be employed to program one or more processors or controllers.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
Fig. 1 is an illustration of a system for fetal monitoring, in accordance with an example.
Fig. 2 is a functional block diagram of a system for fetal monitoring, in accordance with an example.
Fig. 3 is another functional block diagram of a system for fetal monitoring, in accordance with an example.
Fig. 4 is a display screen showing a fetal monitoring dashboard, in accordance with an example.
Fig. 5 is a schematic diagram of a controller of a system for fetal monitoring, in accordance with an example.
Fig. 6 is a flow chart of a method for fetal monitoring, in accordance with an example.

### DESCRIPTION OF EMBODIMENTS

The present disclosure is generally directed to a fetal monitoring system, and more particularly, to systems and methods for capturing and tracking fetal movement. The system is typically embodied as a wearable device with two or more electrodes, a user interface device with at least one input sensor used to track fetal movement, and a processor.

Some hospital fetal monitors offer the option to make use of a clicker for maternal annotation of fetal movements. Similarly, multiple kick-counter mobile device applications are available to register fetal movements. However, integration of either option into a known electrophysiology-based monitoring system would lead to an inconvenient and non-user-friendly solution, since multiple devices would need to be handled by the user. In the embodiment disclosed herein, wherein the fetal monitoring system is a wearable device, e.g., the fetal monitoring system in a single unit comprises the two or more electrodes, the user interface device and the processor, the abovementioned problem is resolved, improving ease of use.

The two or more electrodes are arranged on the user. The user interface device is configured to enable a user to interact with or activate the input sensor upon feeling or observing fetal movement. The processor uses a detection algorithm to generate fetal movement detection data used to track fetal movement based on signals captured by the input sensor. The processor also uses additional detection algorithms to detect additional electrophysiology-based cardiotocography (eCTG) data, such as fetal heart rate and/or uterine activity. The processor then synchronizes and combines the fetal movement data and the eCTG data into a time-aligned dashboard for visualization on a display screen.

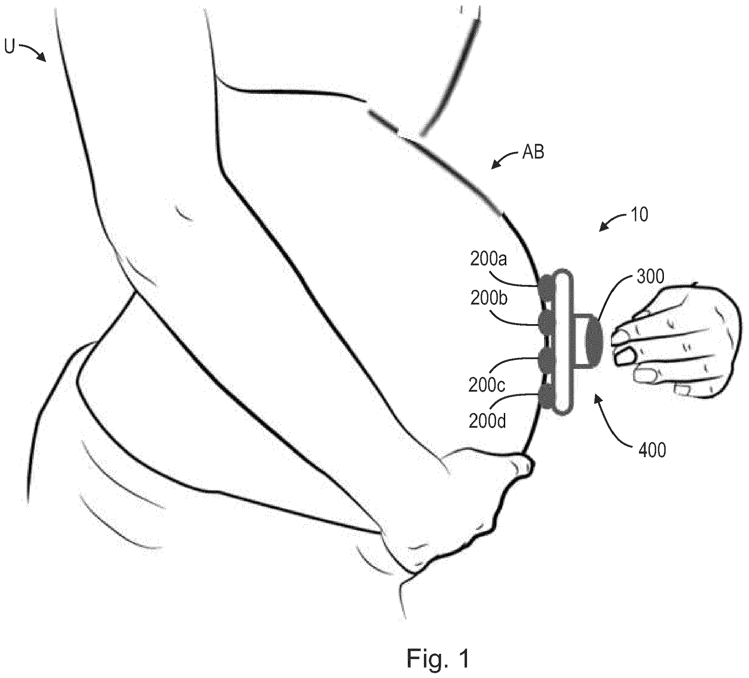

Turning now to the figures, Fig. 1 illustrates a fetal monitoring system 10. In this non-limiting example, the fetal monitoring system 10 is embodied as a wearable, integrated device to be arranged on an abdomen AB of a pregnant user U. The fetal monitoring system 10 includes four electrodes 200a, 200b, 200c, 200b positioned to contact the abdomen AB of the user U. While the fetal monitoring system 10 illustrates four electrodes 200a, 200b, 200c, 200d, any practical number of electrodes 200a, 200b, 200c, 200d may be used. In some examples, the fetal monitoring system 10 may have as few as two electrodes 200, while in some other examples, the fetal monitoring system 10 may have six or more. These electrodes 200a, 200b, 200c, 200d may be arranged in any practical configuration or pattern on the user U. While the four electrodes of 200a, 200b, 200c, 200d shown in Fig. 1 are positioned to contact the abdomen AB, the system 10 may also include other electrodes 200 arranged in other positions on the user U. For example, some electrodes 200 may be arranged on the chest area, back area, or arm area of the user U.

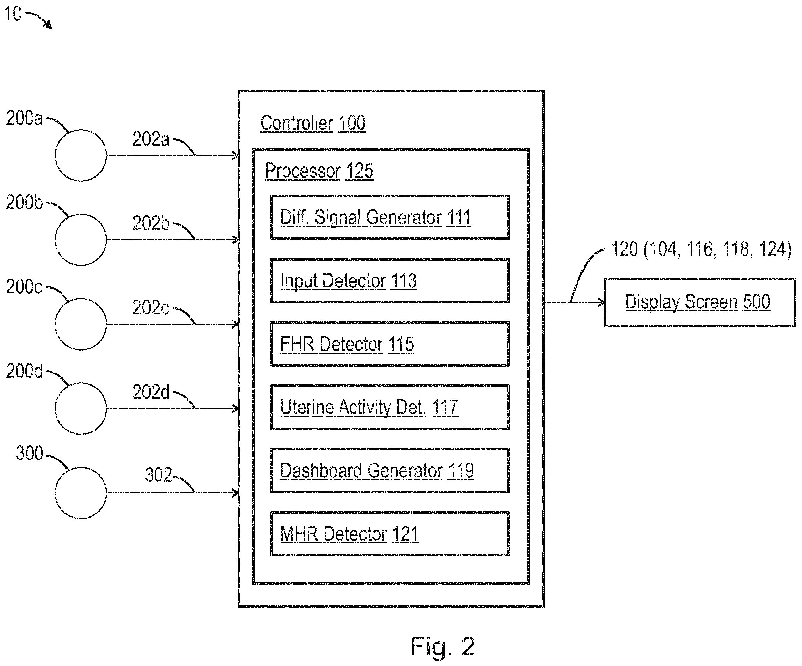

The electrodes 200a, 200b, 200c, 200d of Fig. 1 are each electrically coupled to a controller 100 (see Fig. 2) integrated within the fetal monitoring system 10. As further shown in Fig. 2, each of the electrodes 200a, 200b, 200c, 200d provide the controller 100 with an electrode signal 202a, 202b, 202c, 202b. In some examples, these electrodes 200a, 200b, 200c, 200b are dry electrodes. In other eCTG systems, the controller 100 uses the electrode signal 202a, 202b, 202c, 202b provided by the dry electrodes to measure voltage potential between two areas on the abdomen AB. The measured voltage potentials are then used to track various fetal monitoring characteristics, including fetal heart rate, uterine activity (such as contractions), and maternal heart rate. Further, according to some methods, these dry electrodes may be used to track gross-body fetal movement by tracking the location of the fetal heart relative to the electrodes. However, these methods are unable to detect other types of fetal movements, such as kicks, without additional inputs.

Electrodes 200 for measuring fetal heart rate, uterine activity, and maternal heart rate are commonly arranged on the abdomen AB of the user U to achieve the highest possible signal-to-noise ratio (SNR) of the signals captured by the electrodes 200. However, although it may not be an optimal choice in terms of SNR, the electrodes could 200 still perform these measurements from other positions on the user U. For example, fetal heart rate, uterine activity, and maternal heart rate could be measured with a first electrode 200a arranged on the abdomen, and a second electrode 200b arranged on the back of the user U.

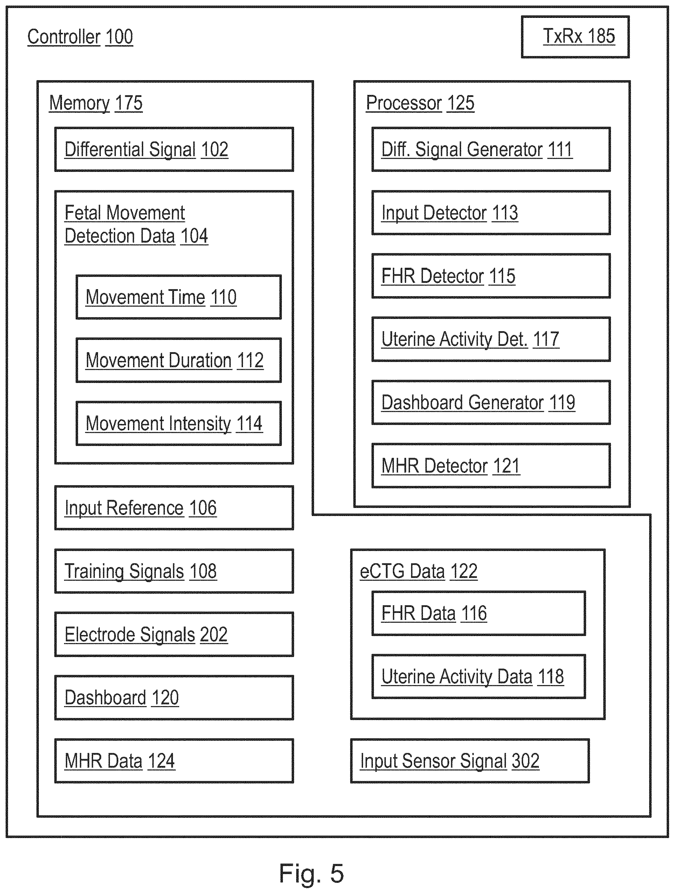

To track fetal movement, the fetal monitoring system 10 further includes a user interface device 400. In the example of Fig. 1, the user interface device 400 is embodied as a housing containing an input sensor 300. The user interface device 400 positions the input sensor 300 such that the input sensor 300 faces outward relative to the abdomen AB of the user U. Accordingly, the user interface device 400 enables the user U to activate or interact with input sensor 300. Upon noticing fetal movement, the user U touches (or nearly touches) the input sensor 300 to register the fetal movement, thus storing a record of the fetal movement in a memory 175 (see Fig. 5) of the controller 100. Thus, the user interface device 400 provides the input sensor 300 as an easy and accessible way to track fetal movement, in addition to fetal heart rate and uterine activity.

In some examples, the input sensor 300 is a dry electrode configured to register fetal movement upon the user U touching the dry electrode. In other examples, the input sensor 300 may be a capacitive bioelectric sensor configured to register fetal movement when the user U brings their finger close to the input sensor 300. The capacitive bioelectric sensor may be particularly useful when the user interface device 400 includes a textile belt to mount the fetal monitoring system 10 to the abdomen AB of the user U. In this example, the material of the textile belt may cover the input sensor 300, preventing the user U from directly (galvanically) contacting the input sensor 300. The textile belt may be made of cloth, elastic, and/or other materials. In these examples, the input sensor 300 is arranged proximate to the abdomen AB of the user U for easy access. While the example of Fig. 1 illustrates a wearable, integrated device including the electrodes 200a, 200b, 200c, 200d, the input sensor 300, and the controller 100, in other examples, these components may be configured as discrete components. In further examples, the input sensor 300 may be embodied as a plurality of sensors, such as several dry electrodes and/or capacitive bioelectric sensors.

The type of user input may also convey additional information regarding the fetal movement. For example, the duration of the user interacting with the input sensor 300 may be indicative of the duration or intensity of the fetal movement. In other examples, multiple successive inputs may be indicative of a particularly intensive fetal movement. A wide variety of input variations and corresponding movement characteristics may be programmed into the controller 100.

Fig. 2 illustrates a functional block diagram of a non-limiting example of a system 10 for fetal monitoring. Generally, the system 10 analyzes data collected via a plurality of electrodes 200a, 200b, 200c, 200d and an input sensor 300 to track fetal movement when detected by the user U. The electrodes 200a, 200b, 200c, 200d provide electrode signals 202a, 202b, 202c, 202d to the controller 100. The input sensor 300 provides an input sensor signal 302 to the controller 100. As described with reference to Fig. 1, the input sensor 300 may be arranged on or within a user interface device 400 to enable a user U to activate or interact with the input sensor 300. The system 10 may also use the collected data to determine additional eCTG information, such as fetal heart rate and/or uterine activity. The fetal movement information and the additional eCTG information may then be provided to a clinician via a dashboard 120 visualized on a display screen 500.

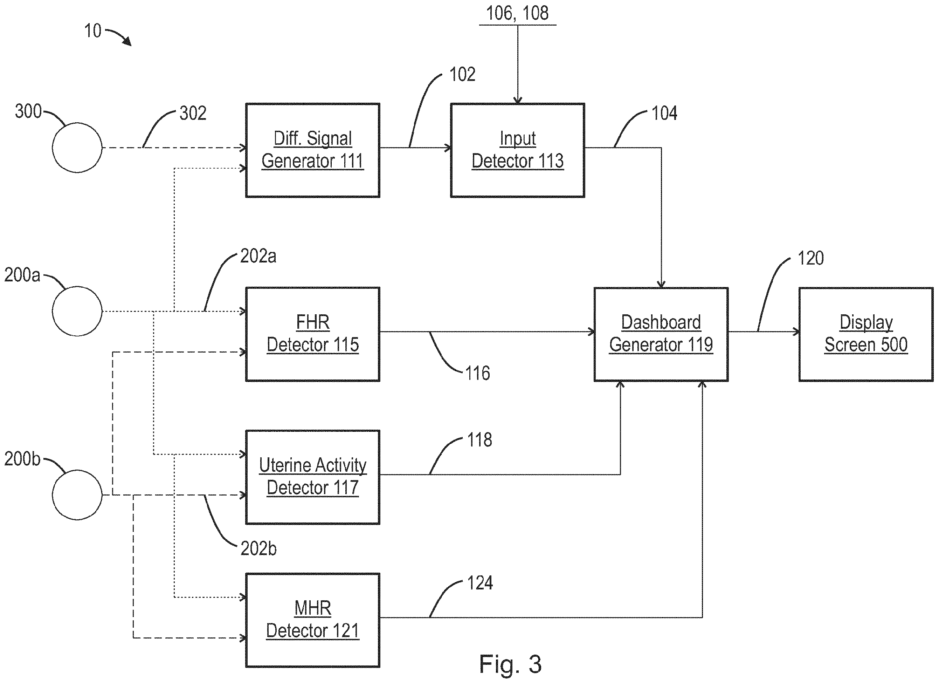

With continued reference to Fig. 2, the system 10 includes a controller 100. The controller 100, shown in more detail in Fig. 5, includes a processor 125, a memory 175, and a transceiver 185. The processor 125 is configured to execute a variety of modules to determine various aspects of the system 10. In the example of Fig. 1, the processor 125 is configured to execute a differential signal generator 111, an input detector 113, a fetal heart rate detector 115, a uterine activity detector 117, a dashboard generator 119, and a maternal heart rate detector 121. These modules are described in further detail with reference to Fig. 3. The memory 175 is configured to store data received by the controller 100 via wired or wireless connection. The memory 175 is further configured to store data generated by the processor 125.

The transceiver 185 is configured to wirelessly transmit data to or wirelessly receive data from various aspects of the system 10. The transceiver 185 may also enable wireless communication with components arranged externally to the system 10. As shown in more detail in the example of Fig. 2, the transceiver 185 may wirelessly transmit data to a display screen 500, such as a dashboard 120 displaying information such as (but not necessarily limited to) fetal movement detection data 104, fetal heart rate data 116, uterine activity data 118, and maternal heart rate data 124.

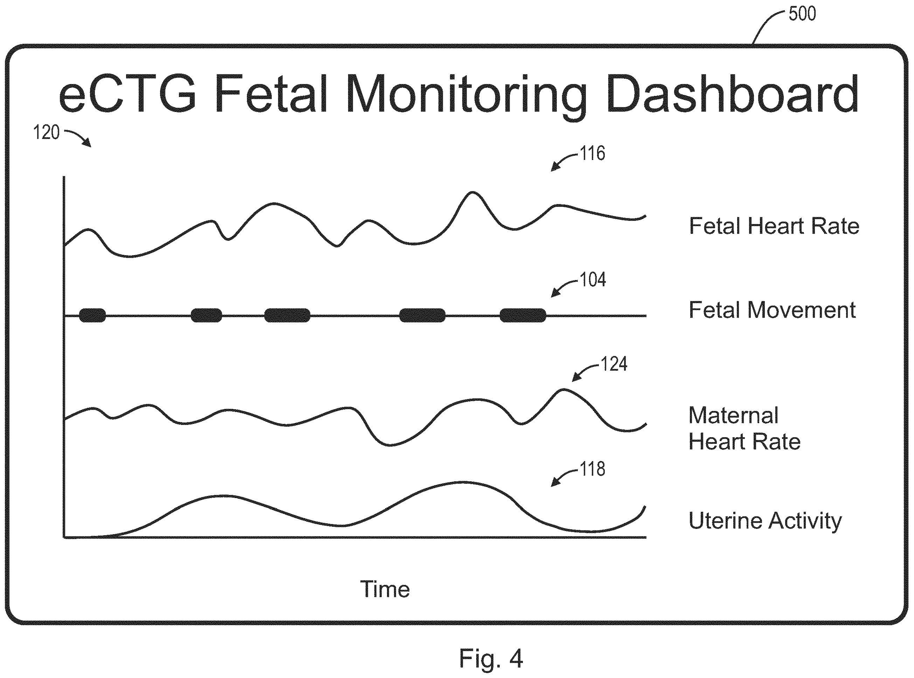

The system 10 may also include a display screen 500. A non-limiting example of the display screen 500 is shown in Fig. 4. In some examples, the display screen 500 may be an analog or digital display to show a dashboard 120 generated by the controller 100 showing various information regarding fetal monitoring, such as fetal movement detection data 104, fetal heart rate data 116, uterine activity data 118, and maternal heart rate data 124. The dashboards 120 may be conveyed to the display screen 500 via wired or wireless connection, such as via the transceiver 185 of the controller 100. The display screen 500 may be any practical type of display, such as a touch screen display. In some examples, the display screen 500 may be a component of the controller 100.

Fig. 3 is another functional block diagram of the system 10 for fetal monitoring. In particular, Fig. 3 illustrates the flow of data between the various modules and programs executed by the processor 125 of the controller 100 to track fetal movements and other fetal activity based on the input sensor 300 capturing a maternal ECG signal when the user feels or observes fetal movement.

As shown in Fig. 3, the system 10 may include two electrodes 200a, 200b and an input sensor 300. In some examples, the system may include more than one input sensor 300. As illustrated in Fig. 1, the two electrodes 200a, 200b, are arranged on the abdomen AB of the user U, while the input sensor 300 may be arranged proximate to the abdomen AB such that the user may touch the input sensor 300 when she feels a fetal movement. However, in other examples, one of the electrodes 200a, 200b may be arranged at a different position on the user U, such as chest area, back area, or arm area. The input sensor 300 may be arranged on or within a user interface device 400 to enable a user U to activate or interact with the input sensor 300 to track fetal movement.

The first electrode 200a and the input sensor 300 are electrically coupled to the differential signal generator 111. The differential signal generator 111 is configured to generate a differential signal 102. The differential signal 102 represents the voltage difference between the input sensor 300 and the first electrode 200a. When the user U touches the input sensor 300, the differential signal 102 will be a maternal electrocardiogram (ECG) signal specific to the user U. Accordingly, to track fetal movement by touching the input sensor 300, the user U should touch the input sensor 300 for a period long enough to capture a full cycle of an adult ECG signal, typically a couple of seconds. When the input sensor 102 is not touched, the differential signal 102 is very electrically noisy due to picking up electromagnetic (EM) interference, such as 50 Hz and 60 Hz interference, from nearby electronic devices. As previously discussed, the input sensor 300 may be a dry electrode. In other examples, the input sensor 300 may be a capacitive bioelectric sensor. A capacitive bioelectric sensor may be preferrable in embodiments where the user U is unable to directly touch the input sensor 300, such as if the input sensor 300 is covered by a textile belt to secure the fetal monitoring system 10 in place. In some examples, the first electrode 200a may be arranged on the abdomen AB of the user U for optimized SNR. However, in other examples, the first electrode 200a may be arranged on the chest area, back area, or arm area of the user U.

The differential signal 102 is provided to the input detector 113. The input detector 113 analyzes the differential signal 102 to determine if a user U has touched or interacted with the input sensor 300 to track fetal movement. In one example, the input detector 113 may use a detection algorithm to determine if the differential signal 102 contains an ECG signal, or is just noise. The detection algorithm may use the Pan-Tompkins algorithm to detect QRS complexes to verify the presence of an ECG waveform. Alternatively, the detection algorithm may rely on ECG wavelet transforms or other ECG decomposition techniques to detect an ECG signal within the differential signal 102.

In further examples, the detection algorithm may compare the differential signal 102 with an input reference signal 106 to determine if the user U has touched or interacted with the input sensor 300. In some examples, the input reference signal 106 is a generic ECG signal. In other examples, the input reference signal 106 is calibrated for the user U based on a series of training differential signals 108. The training differential signals 108 are the result of the user U touching the input sensor 300 prior to using the input sensor 300 to track fetal movement. In a preferred example, the training differential signal 108 may correspond to the user U touching the input sensor 300 with the index fingers of both their left and right hands separately to capture the ECG signals corresponding to each finger, as their different hands will have different ECG morphologies.

An advantage of the fetal movement detection data being generated based on the differential signal 102 and/or the input reference signal 106, is prevention of registering fetal movements by mistake, which lead to fetal movement false positives. For example, undesired triggers originating from external motion, ambient noise and accidentally touching the input sensor 300 can be prevented. As a result, accuracy of the fetal monitoring system 10 is improved.

The input detector 113 uses the aforementioned algorithms and references to generate fetal movement detection data 104 based on the differential signal 102. The fetal movement detection data 104 represents the incidents of fetal movement registered by the user U via the input sensor 300. In some examples, the fetal movement detection data 104 simply records each incident of registered fetal movement. In other examples, the fetal movement detection data 104 may incorporate additional information regarding the fetal movement. For example, the fetal movement detection data 104 may track movement time 110 (the time the movement was registered), movement duration 112 (how long the fetus was moving), and movement intensity 114. For example, movement time 110 may be automatically generated based on an internal clock of the controller 100. The movement duration 112 and the movement intensity 114 may correspond to how the user U touched the input sensor 300. For example, the movement duration 112 may correspond to the length of time a finger of the user U remains in contact with the input sensor 300, with longer contact corresponding to longer movement duration 112. Similarly, multiple successive inputs may be indicative of a single high intensity movement, with more inputs corresponding to higher movement intensity 114. In even further examples, the fetal movement detection data 104 may be configured to track fetal movement type. For example, different inputs could correspond to different types of fetal movement, such as kicks, hiccups, rolls, turns, and/or twists.

As shown in Fig. 3, system 10 may also include a fetal heart rate detector 115 and a uterine activity detector 117. The fetal heart rate detector 115 is configured to generate fetal heart rate data 116 based on inputs from the first and second electrodes 200a, 200b using known eCTG techniques. The fetal heart rate data 116 includes the fetal heart rate of the fetus. Similarly, the uterine activity detector 117 is configured to generate uterine activity data 118 based on inputs from the first and second electrodes 200a, 200b using known eCTG techniques. The uterine activity data 118 may include information regarding uterine contractions. In some examples for measuring fetal heart rate data 116 and uterine activity data 118, the first and second electrodes 200a, 200b are both arranged on the abdomen AB of the user U for optimized SNR. In other examples, the first electrode 200a is arranged on the abdomen AB, while the second electrode 200b is positioned on another part of the user U, such as a chest area, back area, or arm area.

The system 10 may also include a maternal heart rate detector 121. The maternal heart rate detector 121 is configured to generate maternal heart rate data 124 based on inputs from the first and second electrodes 200a, 200b using known techniques. The maternal heart rate data 124 includes the maternal heart rate of the user U. In some examples for measuring maternal heart rate data 124, the first and second electrodes 200a, 200b are both arranged on the abdomen AB of the user U for optimized SNR. In other examples, the first electrode 200a is arranged on the abdomen AB, while the second electrode 200b is positioned on another part of the user U, such as a chest area, back area, or arm area.

In the non-limiting example of Fig. 3, the system 10 may include a dashboard generator 119. The dashboard generator 119 receives the fetal movement detection data 104 from the input detector 113, the fetal heart rate data 116 from the fetal heart rate detector 115, the uterine activity data 118 from the uterine activity detector 117, and the maternal heart rate data 124 from the maternal heart rate detector 121. The dashboard generator 119 may then synchronize and combine this data into a time-aligned fetal monitoring dashboard 120. The dashboard 120 is then provided to the display screen 500 for display.

In further examples, the input sensor 300 may not be a touch sensor, but rather a different type of sensor. For example, the input sensor 300 could be one or more microphones configured to capture spoken audio from the user U. In this example, the user U could speak a known word or phrase as an "input signature" to track fetal movement. The input detector 113 could then analyze the captured audio to determine if the captured audio corresponds to, e.g., matches, the input signature. In another example, the input sensor 300 could be a fingerprint sensor configured to capture a fingerprint from the user U. In this example, the "input signature" could be one or more previously captured fingerprints of the user U. The input detector 113 could then analyze the captured fingerprint to determine if it corresponds to, e.g., matches, the input signature. Other types of non-touch sensors could be used in connection with a variety of user interface devices 400.

In additional examples, the input sensor 300 could be used to track other information rather than fetal movement. For example, the input sensor 300 could track information requiring manual annotation, such as the amount of water drank in a specific time period, or pregnancy symptom information.

In even further examples, touching the input sensor 300 could activate an annotation mode in a secure manner. Once in annotation mode, the user U could tap on another input control (such as an accelerometer) to annotate a parameter of interest.

Fig. 4 illustrates an example of the display screen 500 of Fig. 3. The display screen 500 of Fig. 4 may any type of visual display, such as a touch screen. The display screen 500 may be embedded in an existing control station or console, or it may be a part of a portable device, such as a tablet computer or mobile device. The example display screen 500 of Fig. 4 displays a non-limiting example of an eCTG fetal monitoring dashboard 120. As shown in this example, the dashboard 120 displays, from top-to-bottom, the fetal heart rate data 116, the fetal movement detection data 104, the maternal heart rate data 124, and the uterine activity data 118 in a time-aligned manner. As can be seen in Fig. 4, the fetal movement data 104 is represented with a series of dashes of varying length. In some examples, the longer dashes are indicative of fetal movement of longer duration, as represented by the movement duration 112 of the fetal movement detection data 104. In other examples, longer and/or thicker dashes are indicative of fetal movement of stronger intensity, as represented by the movement intensity 114 of the fetal movement detection data 104.

Fig. 5 schematically illustrates the controller 100 previously depicted in Fig. 1. The controller 100 includes the processor 125, the memory 175, and the transceiver 185. The memory 175 is configured to store the differential signal 102, the fetal movement detection data 104, the input reference 106, the training signals 108, the eCTG data 122 (such as the fetal heart rate data 116 and the uterine activity data 118), the fetal monitoring dashboards 120, the maternal heart rate data 124, the electrode signals 202, and the input sensor signal 302. The fetal movement detection data 104 may include movement time 110, movement duration 112, and movement intensity 114. The processor 125 is configured to execute the differential signal generator 111, the input detector 113, the fetal heart rate detector 115, the uterine activity detector 117, the dashboard generator 119, and the maternal heart rate detector 121.

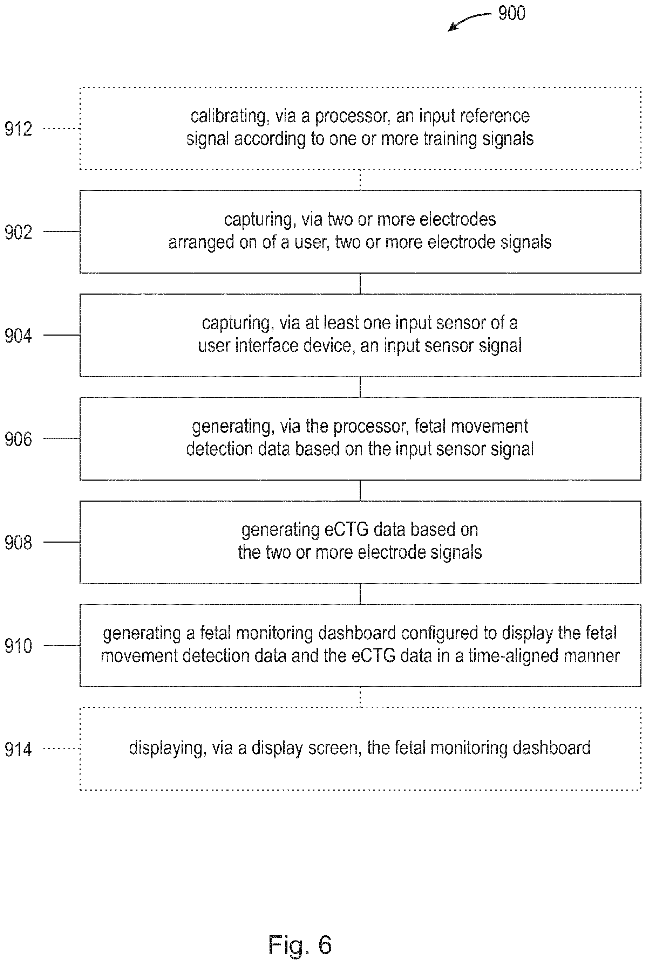

Fig. 6 is a flow chart of a method 900 for fetal monitoring. Referring to Figs. 1-6, the method 900 includes, in step 902, capturing, via two or more electrodes 200 arranged on a user U, two or more electrode signals 202.

The method 900 further includes, in step 904, capturing, via at least one input sensor 300 of a user interface device 400, an input sensor signal 302.

The method 900 further includes, in step 906, generating, via a processor 125, fetal movement detection data 104 based on the input sensor signal 302. In some examples, the fetal movement detection data 104 is generated further based on an input reference signal 106.

The method 900 further includes, in step 908, generating eCTG data 122 based on the two or more electrode signals 202.

The method 900 further includes, in step 910, generating a fetal monitoring dashboard 120 configured to display the fetal movement detection data 104 and the eCTG data 122 in a time-aligned manner.

According to an example, the method 900 further includes, in optional step 912, calibrating, via the processor 125, the input reference signal 106 according to one or more training differential signals 108.

According to an example, the method 900 further includes, in optional step 914, displaying, via a display screen 500, the fetal monitoring dashboard 120.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

The above-described examples of the described subject matter can be implemented in any of numerous ways. For example, some aspects may be implemented using hardware, software, or a combination thereof. When any aspect is implemented at least in part in software, the software code can be executed on any suitable processor or collection of processors, whether provided in a single device or computer or distributed among multiple devices/computers.

The present disclosure may be implemented as a system, a method, and/or a computer program (product) at any possible technical detail level of integration. A computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some examples, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions may be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant may be entitled.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. In the device claim enumerating several means, several of these means may be embodied by one and the same item of hardware. Measures recited in mutually different dependent claims may advantageously be used in combination.

## Claims

1. A fetal monitoring system (10), comprising:
at least two electrodes (200) configured to be arranged on a user (U) and to capture two or more electrode signals (202);
a user interface device (400) comprising at least one input sensor (300), wherein the input sensor (300) is configured to capture an input sensor signal (302); and
a processor (125) configured to:
generate fetal movement detection data (104) based on the input sensor signal (302);
generate electrophysiology-based cardiotocography (eCTG) data (122) based on the two or more electrode signals (202); and
generate a fetal monitoring dashboard (120) configured to display the fetal movement detection data (104) and the eCTG data (122) in a time-aligned manner.

2. The fetal monitoring system (10) of claim 1, wherein the fetal movement detection data (104) is generated further based on a differential signal (102), and wherein the differential signal (102) is generated based on the input sensor signal (302) and one of the two or more electrode signals (202).

3. The fetal monitoring system (10) of claim 2, wherein the differential signal (102) is a maternal electrocardiogram signal corresponding to the user (U).

4. The fetal monitoring system (10) of one or more of claims 1-3, wherein the processor (125) is further configured to generate maternal heart rate data (124) based on the two or more electrode signals (202), and wherein the fetal monitoring dashboard (120) is further configured to display the maternal heart rate data (124).

5. The fetal monitoring system (10) of one or more of claims 1-4, wherein the fetal movement detection data (104) is generated further based on an input reference signal (106).

6. The fetal monitoring system (10) of claim 5, wherein the input reference signal (106) is a predefined electrocardiogram signal.

7. The fetal monitoring system (10) of claim 5, wherein the input reference signal (106) is unique to the user (U), and the processor (125) is further configured to calibrate the input reference signal (106) according to one or more training signals (108).

8. The fetal monitoring system (10) of one or more of claims 1-7, wherein the fetal movement detection data (104) comprises movement time (110), movement duration (112), and movement intensity (114).

9. The fetal monitoring system (10) of one or more of claims 1-8, wherein the eCTG data (122) comprises fetal heart rate data (116) and/or uterine activity data (118).

10. The fetal monitoring system (10) of one or more of claims 1-9, further comprising a display screen (500) configured to display the fetal monitoring dashboard (120).

11. The fetal monitoring system (10) of one or more of claims 1-10, wherein the at least one input sensor (300) is an additional electrode, a capacitive bioelectric sensor, a microphone, or a fingerprint sensor.

12. A method (900) for fetal monitoring, comprising:
capturing (902), via two or more electrodes (200) arranged on a user (U), two or more electrode signals (202);
capturing (904), via at least one input sensor (300) of a user interface device (400), an input sensor signal (302);
generating (906), via a processor (125), fetal movement detection data (104) based on the input sensor signal (302);
generating (908) electrophysiology-based cardiotocography (eCTG) data (122) based on the two or more electrode signals (202); and
generating (910) a fetal monitoring dashboard (120) configured to display the fetal movement detection data (104) and the eCTG data (122) in a time-aligned manner.

13. The method (900) of claim 12, wherein the fetal movement detection data (104) is generated further based on an input reference signal (106).

14. The method (900) of claim 13, further comprising calibrating (912), via the processor (125), the input reference signal (106) according to one or more training signals (108).

15. The method (900) of one or more of claims 12-14, further comprising displaying (914), via a display screen (500), the fetal monitoring dashboard (120).
